# EUROPÄISCHE PATENTSCHRIFT

(11) **EP 4 233 547 B1**
(45) Veröffentlichungstag und Bekanntmachung des Hinweises auf die Patenterteilung: **25.06.2025**
(21) Anmeldenummer: 22216673.8
(22) Anmeldetag: 24.12.2022
(51) Int. Cl.: A01N 65/22, A01N 63/10, A01P 1/00

(54) **ZUSAMMENSETZUNG MIT WIRKUNG GEGEN BORRELIEN UND VERFAHREN ZU IHRER HERSTELLUNG**
COMPOSITION WITH ANTI-BORRELIA EFFECT AND METHOD FOR THE PRODUCTION THEREOF
COMPOSITION À EFFET ANTI-BORRELIA ET SON PROCÉDÉ DE FABRICATION

(43) Veröffentlichungstag der Anmeldung: 30.08.2023
(73) Patentinhaber: Patentpool Target GmbH, 80331 München (DE)
(72) Erfinder: Burda, Renate, 82054 Sauerlach (DE); Mörth-Kretschmer, Jenny, 21244 Buchholz in der Nordheide (DE); Sand, Elisabeth, 91567 Rauenzell/Herrieden (DE); Weickmann, Dirk, 91567 Rauenzell/Herrieden (DE)
(74) Vertreter: Reich, Jochen

(56) Entgegenhaltungen:
- EP-A2- 1 754 486
- US-B1- 10 238 619
- DEMIRCI BETUL ET AL: "Effects of Salvia essential oils on the chorioallantoic membrane (CAM)", PHARMACEUTICAL BIOLOGY, SWETS AND ZEITLINGER, LISSE, NL, vol. 43, no. 8, 1 November 2005 (2005-11-01), pages 666 - 671, XP009170557, ISSN: 1388-0209, DOI: 10.1080/13880200500383397
- ANNA GOC ET AL: "Anti-borreliae efficacy of selected organic oils and fatty acids", BMC COMPLEMENTARY AND ALTERNATIVE MEDICINE, BIOMED CENTRAL LTD, LONDON, UK, vol. 19, no. 1, 4 February 2019 (2019-02-04), pages 1 - 11, XP021269858, DOI: 10.1186/S12906-019-2450-7
- OZKAN G ET AL: "Study on chemical composition and biological activities of essential oil and extract from Salvia pisidica", LWT- FOOD SCIENCE AND TECHNOLOGY, ACADEMIC PRESS, UNITED KINGDOM, vol. 43, no. 1, 1 January 2010 (2010-01-01), pages 186 - 190, XP026626291, ISSN: 0023-6438, [retrieved on 20090627], DOI: 10.1016/J.LWT.2009.06.014
- KÜSTER TATIANA ET AL: "Voluntary Ingestion of Antiparasitic Drugs Emulsified in Honey Represents an Alternative to Gavage in Mice", JOURNAL OF THE AMERICAN ASSOCIATION FOR LABORATORY ANIMAL SCIENCE, 1 March 2012 (2012-03-01), U.S.A., pages 219 - 223, XP093045323, Retrieved from the Internet <URL:https://www.ncbi.nlm.nih.gov/pmc/articles/PMC3314525/pdf/jaalas2012000219.pdf> [retrieved on 20230509]
- OZKAN AYSUN ET AL: "Antitumoral and antioxidant effect of essential oils and in vitro antioxidant properties of essential oils and aqueous extracts from Salvia pisidica", vol. 65, no. 6, 1 December 2010 (2010-12-01), DE, pages 990 - 996, XP093045420, ISSN: 0006-3088, Retrieved from the Internet <URL:https://link.springer.com/content/pdf/10.2478/s11756-010-0108-5.pdf?pdf=button> DOI: 10.2478/s11756-010-0108-5
- IHSAN SAAD A ET AL: "Chemical Analysis and Biological Activities of Salvia lavandulifolia Vahl. Essential Oil", JOURNAL OF BIOLOGY, AGRICULTURE AND HEALTHCARE, 1 January 2017 (2017-01-01), pages 71 - 78, XP093045527, Retrieved from the Internet <URL:https://core.ac.uk/download/pdf/234662242.pdf> [retrieved on 20230510]

## Beschreibung

In den 1980er-Jahren wurde FSME und FME beschrieben. Die Frühsommer-Meningoenzephalitis ist eine durch das FSME-Virus ausgelöste Erkrankung, die mit ähnlichen Symptomen wie bei der Borreliose auftritt, also Fieber und partiell einer Meningoenzephalitis, der Entzündung von Gehirn und Hirnhäuten.

Borreliose wird von Bakterien und nicht wie FSME durch Viren ausgelöst. Die Übertragungsgefahr bestand "damals" nur bei jedem 10.000 oder 100.000 Zeckenbiss/ -stich. Heutzutage kann man davon ausgehen, dass bei jedem Zeckenstich Borrelien übertragen werden. Die Gefährlichkeit der Borrelien und der Zusammenhang mit rheumatischen Erkrankungen wurde lange nicht erkannt, auch weil die Zeit zwischen einem Zeckenstich und dem Ausbruch der Krankheit bis zu 40 Jahre dauern kann. Meist vergehen Monate oder Jahre nach einem Zeckenbiss bis zum Ausbruch der Krankheit. Vermutlich wegen des allgemein schwächeren Immunsystems der "modernen" Bevölkerung und der allgemeinen Belastung der gesamten Umwelt mit Schadstoffen kommt es heute oft viel schneller zum Ausbruch der Krankheit mit zudem schwerwiegenderen Verläufen. Wir sind der Überzeugung, dass die meisten vor allem früher bei der ländlichen Bevölkerung aufgetretenen und auch heute noch auftretenden rheumatischen Beschwerden tatsächlich auf Borrelien-Infektionen zurückzuführen sind.

Borreliose äußert sich anfangs mit unspezifischen Allgemeinsymptomen wie Fieber, Kopfschmerzen oder Magen-Darm-Beschwerden. Die Hautrötung (Erythema migrans oder Wanderröte) ist ein eindeutiges Symptom für eine Borrelieninfektion, die aber nicht bei allen Borreliose-Infektionen auftritt. Ein Fehlen der Wanderröte nach einem Zeckenbiss bedeutet nicht automatisch, dass keine Borreliose-Infektion erfolgt ist.

Symptome wie Grippe, Fieber und Kopfschmerzen erschweren das Erkennen der Krankheit. Es kann zu einem Befall der Organe, der Gelenke und Muskeln sowie des zentralen und peripheren Nervensystems kommen. Das Immunsystem ist in diesem Stadium oft nicht mehr in der Lage, die Infektionen zu bewältigen. Borrelien halten sich nur kurz im Blut auf und setzen sich im Bindegewebe und in Zellwänden fest, sie leben sozusagen versteckt. Hier sind sie schwer zu erkennen und deshalb ist eine länger andauernde Behandlung nötig.

Stand der Technik bei der Behandlung:
Wenn die Infektion noch nicht weit fortgeschritten ist kann mit Antibiotika behandelt werden. Am effektivsten zeigt sich hier die Behandlung mit reinem Penicillin G (C16H18N2O4S). Ob andere Antibiotika einen dauerhaften und nachhaltigen Effekt haben ist aktuell nicht bestätigt und im Moment noch anzuzweifeln. Die Behandlung mit Penicillin kann jedoch, wenn zufällig der richtige Zeitpunkt (bevor sich die Bakterien im Gewebe "verstecken") erkannt wird, hilfreich sein; jedoch diesen Zeitpunkt zu erkennen ist annähernd unmöglich. Ab dem Zeitpunkt der Übertragung des Erregers kann es nach einer unterschiedlichen Inkubationszeit zu einer Lokalinfektion der Haut kommen, die mit einer Hautrötung an der Stichstelle sichtbar wird. Die Inkubationszeit kann in manchen Fällen mehrere Jahre dauern, weshalb die damit verbundenen Gelenkschmerzen lange Zeit für rheumatische Beschwerden gehalten wurden und erfolglos mit Cortison-Salbe behandelt wurden. Wir sind der Überzeugung, dass die meisten vor allem früher bei der ländlichen Bevölkerung aufgetretenen und auch heute noch auftretenden rheumatischen Beschwerden auf Borrelien zurückzuführen sind.

Probleme bei Diagnose und Therapie:
Entgegen der herkömmlichen Auffassung können sich Borrelien sehr schnell ausbreiten. Einen absolut vorhersagbaren Verlauf, wie die klassische Einteilung in drei Stadien nahelegt, gibt es für die Borreliose nicht, der Ablauf entwickelt sich nicht gesetzmäßig. Passt das Beschwerdebild des Betroffenen nicht in die schulmedizinisch klassische Einteilung der Borreliose, so wird Borreliose oft ausgeschlossen und die Symptome werden falsch behandelt. Eine Borrelien-Infektion (Borreliose) hält sich an kein Schema! Wie sich eine Borreliose entwickelt, lässt sich nicht vorhersagen. Die Symptomatik kann sich schnell und schwerwiegend entwickeln oder es kommt zu einer schleichenden und langsamen Manifestation.

Wenn Borreliose nicht rechtzeitig behandelt wird, kann es zu einer chronischen Infektion kommen. Das heißt, die Krankheit kehrt immer wieder oder verschlechtert sich zunehmend. Selbst jahrelange symptomfreie Latenzzeiten mit anschließender Reaktivierung sind möglich. Die Erreger lösen verschiedene Krankheitsbilder aus. Meist sich die Gelenke betroffen, es kann auch zu Herzproblemen und Gefäßentzündugen führen. Auch Mischformen der Erkrankungen sind möglich. Viele Borreliose-Patienten klagen über Erschöpfungszustände und chronische Müdigkeit, die durch ausreichend Schlaf nicht ausgeglichen werden kann.

Ein für Menschen geeigneter Borreliose-Impfstoff ist in nächster Zukunft nicht zu erwarten. In den USA wurde ein entsprechender Impfstoff 2002 wegen angeblicher Impfkomplikationen wieder vom Markt genommen. Dieser Impfstoff war allerdings nur für den amerikanischen Erregerstamm geeignet und somit in Europa nicht anwendbar. Klinische Studien zu einem weiteren Impfstoff (VLA15) laufen in den USA und Europa.

Einzig echt wirksames Antibiotikum ist rein biologisches Penicillin. Dieses muss gemäß unseren Erfahrungen spätestens sechs Wochen nach der Infektion appliziert werden. Dies geschieht nur in den allerwenigsten Fällen.

### Borreliose aktuell:

Neuste Studien zeigen, dass heutzutage rund jeder 7. Mensch weltweit bereits mindestens 1x mit Borreliose infiziert worden ist. Nach einer im BMJ Global Health publizierten Studie, weist Mitteleuropa mit 20% die höchste Infektionsrate auf. Somit ist es Gegenstand dieser Erfindung eine wirksame Therapie gegen Borreliose zu entwickeln. Mit der Patentschrift WO2007060252A3 haben wir bereits seit 2006 ein Therapeutikum gegen Borreliose welches auch im Rahmen zulässiger ärztlicher Heilversuche positiv bewertet wurde. Hier fanden und entwickelten wir Wirkstoffe aus Gesamtgiftcocktails verschiedener Myriapoda. Nachteil dieser Behandlungsmöglichkeit ist die verhältnismäßig schlechte Verfügbarkeit der Wirkstoffe. Vorliegend vorgeschlagen wird eine Behandlungsmöglichkeit der Borreliose bei bester Verfügbarkeit der Wirkstoffe. Auch Campheröl und das Öl von *Salvia triloba* zeigten einen antimikrobiellen Effekt gegenüber *Borrelia burgdorferii* und *Borrelia garinii.* Dieser Effekt wird vom Öl der Echten Kamille und von Zimtöl noch deutlich übertroffen (A. Goc et al. BMC Complementary and Alternative Medicin 2019, 19, 1-11). Auch Mischungen verschiedener Pflanzenextrakte und Öle sollen einen Effekt gegenüber *Borrelia* sp. besitzen (US10238619).

Es ist somit eine Aufgabe der vorliegenden Erfindung ein Verfahren zur Herstellung einer Zusammensetzung mit Wirkung gegenüber Borrelien sowie die Zusammensetzung vorzuschlagen.

Die Aufgabe wird durch die unabhängigen Ansprüche gelöst. Weitere vorteilhafte Ausgestaltungen sind in den Unteransprüchen angegeben.

Erfindungsgemäße Zusammensetzung und Herstellung:
Die Erfinder fanden nun in einem Salbei, die Salvia pisidica Boiss. & Heldr.. Substanzen die Borrelia spp. und Yersinia spp. in vitro und in vivo abtöten können. Bislang geht man davon aus, daß ein Methanol-Extrakt, bzw. das Öl von Silvia pisidica antimikrobielle Effekte gegen gram (+) Bakterien aufweist. Gegen gram (-) Bakterien ist kaum eine Wirkung nachweisbar. Unsere bisherigen Versuche bestätigen diese aktuelle wissenschaftliche Auffassung.

Die Erfinder fanden nun heraus, dass ein Ethanol-Extrakt der frischen Blätter der Salvia pisidica der in Honig eingearbeitet wird gram (-) Bakterien, speziell Borrelia spp. und/oder Yersinia spp. abtöten kann.

Somit eignet sich ein in Honig eingearbeiteter ethanolischer Extrakt der Salvia pisidica zur Therapie von Lyme-disease.

Weltweit sind etwa 900 Arten Salbei bekannt. Viele haben pharmazeutischmedizinisch interessante Substanzen. Nur wenige Arten wurden bislang untersucht. Wir untersuchten Extrakte der folgenden Salbeiarten hinsichtlich ihrer borrelienabtötenden Wirkung:
Salvia mellifera
Salvia nevadensis
Salvia fruticosa
Salvia cinerea
Salvia pisidica
Salvia lavandulifolia

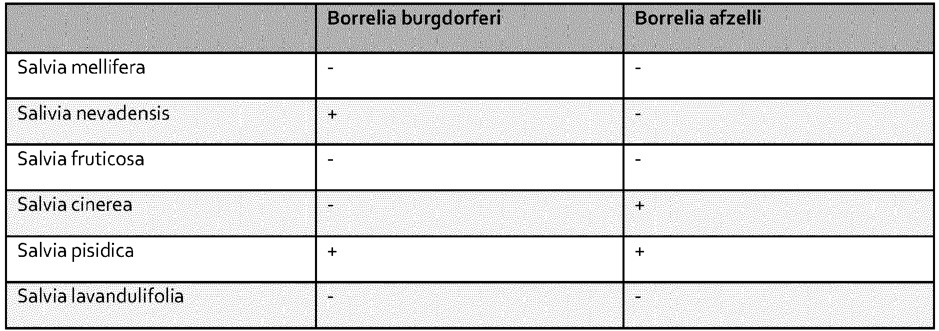

Nur Extrakte von Salvia pisidica zeigten einen nachweislichen erfindungsgemäßen Effekt gegen Borrelien:
Die erfindungsgemäße Zusammensetzung wird gemäß einem Aspekt der vorliegenden Erfindung auf die folgende Weise hergestellt:
a) Es wird eine bestimmte Menge Blätter, bevorzugt frische Blätter, der Salvia pisidica in eine bestimmte Menge 96%iges Ethanol, bzw. bevorzugt 100%iges Ethanol eingebracht.
b) Das Extraktionsgemisch gemäss a) wird mindestens 24 Stunden bei Temperaturen von bevorzugt 25 Grad Celsius stehen gelassen. Anschließend werden die Blätter abfiltriert.
c) Dieser Wirkextrakt aus b) wird in 150 mL verflüssigten Bioblütenhonig unter ständigem Rühren eingearbeitet.

Kultur der Salvia pisidica zur optimalen Wirkstoffsynthese:
Damit Pflanzen, vor allem die für die erfindungsgemäße Verwendung benötigte Art Salvia pisidica relevante Mengen ihrer Wirkstoffe produzieren kann, ist es wichtig die richtigen Kultursubstrate/Böden für die Pflanzen zu haben.

Die Erfinder fanden heraus, dass für die Salvia pisidica nicht nur ein durchlässiger Boden vonnöten ist, sondern dass die Art zur Wirkstoff-Synthese einen kalkhaltigen Boden benötigt. Bevorzugt verwenden wir Kalkerde von flora montana mit ca. 20% verwitternden Kalkstein.

Mehr als 35 verschiedene Komponenten lassen sich im Ethanol-Extrakt der Salvia pisidica nachweisen. Gallensäure-Äquivalente, Rutin-Äquivalente und Catechin-Äquivalente können durch die richtige Substratverwendung gesteigert werden. Eine Kultur in Pflanzengefäßen ist effizienter als eine solche direkt im Boden. Für die Erfindungsgemäße Anwendung ist es wichtig, die frischen Blätter der Pflanzen ohne Stängel zu verwenden. Wirkungen konnten wir beobachten bei folgenden Konzentrationen:

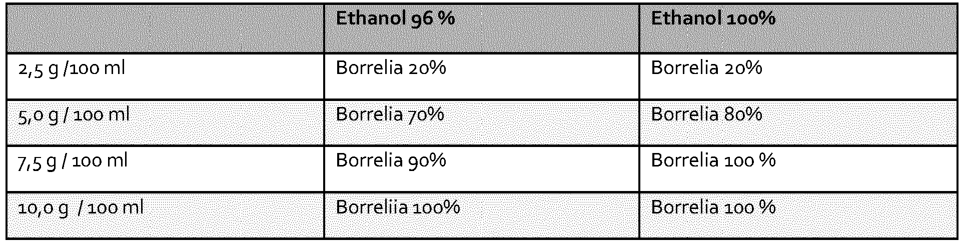

Somit sind die höheren Konzentrationen und die Auszüge mit 100%igem Ethanol am effektivsten.

### Darreichungsformen:

Es wird hiermit beschrieben, jedoch nicht beansprucht, dass die Extrakte oral eingenommen werden können. Die Wirkstoffaufnahme geschieht über den Verdauungstrakt. Der ethanolische Auszug ist besonders gut in Honig lösbar, so ist neben der direkten, puren Einnahme des ethanolischen Extraktes auch eine solche gelöst in Honig sinnvoll.

Die Aspekte der vorliegenden Erfindung sind eine Zusammensetzung gemäss der Ansprüche 1-4 sowie ein Verfahren zur Herstellung einer Zusammensetzung gemäss der Ansprüche 1-4 gemäss der Ansprüche 5-9.
- Zusammensetzung enthaltend einen ethanolischen Auszug der Blätter der Salvia pisidica aufweisend:
   - einen Auszug von Blättern der Salvia pisidica; mit zumindest 96%igem Ethanol; und
   - Zumindest Bienenhonig.
- Zusammensetzung enthaltend einen ethanolischen Auszug der Blätter der Salvia pisidica mit folgenden Bestandteilen:
   a) einen Auszug von zumindest jungen Blättern der Salvia pisidica mit zumindest 96%igem Ethanol
   und b) Zumindest Bienenhonig
- Zusammensetzung enthaltend einen ethanolischen Auszug der Blätter der Salvia pisidica mit folgenden Bestandteilen:
   a) einen Auszug von Blättern der Salvia pisidica mit zumindest 100%igem Ethanol
   und b) Zumindest Bienenhonig
- Zusammensetzung enthaltend einen ethanolischen Auszug der Blätter der Salvia pisidica nach einem der Ansprüche 1 bis 4, dadurch gekennzeichnet das der Bienenhonig die Qualität von Bioblütenhonig aufweist.
- Verfahren zur Herstellung einer Zusammensetzung nach einem der Ansprüche 1 bis 4, dadurch gekennzeichnet das folgende Maßnahmen getroffen werden:
   - Es wird eine bestimmte Menge Blätter, bevorzugt frische Blätter, der Salvia pisidica in eine bestimmte Menge 96%iges Ethanol, bzw. bevorzugt 100%iges Ethanol eingebracht.
   - Das Extraktionsgemisch gemäss
      a) wird mindestens 24 Stunden bei Temperaturen von bevorzugt 25 Grad Celsius stehen gelassen. Anschließend werden die Blätter abfiltriert.
   - Dieser Wirkextrakt aus b) wird in 150 mL verflüssigten Bioblütenhonig unter ständigem Rühren eingearbeitet.

Die Blätter der Salvia pisidica, zur erfindungsgemäßen Verwendung, sind am gehaltvollsten mit einer Länge von etwa 1,7 bis 2,1 cm und einer Breite von 0,9 bis 1,3 cm (einzelne Fieder 0,3 bis 0,5 cm). Von der Salvia pisidica werden frische Blätter, nicht unbedingt junge Blätter verwendet. Das heißt es sind Blätter direkt von der Pflanze, die direkt nach dem Ernten verarbeitet werden sollten, bzw. spätestens nach 50 Stunden. Direkt heißt hierbei ohne weitere Verarbeitungsschritte. Wenn sie nicht direkt verarbeitet werden sollten sie dunkel und bei Temperaturen von 15 bis 22 Grad Celsius lagern. Frische, junge, hellgrüne Blätter, zur erfindungsgemäßen Verwendung, haben noch mehr Wirkstoff. Diese Blätter sind bis zu 30 Tage alt - je nach Standort der jeweiligen Pflanze.

## Patentansprüche

1. Zusammensetzung aufweisend:
- einen mit mindestens 96%igem Ethanol hergestellten Auszug aus Blättern der der Pflanze Salvia pisidica, welche auf durchlässigem kalkhaltigen Boden gewachsen ist; und
- Bienenhonig.

2. Zusammensetzung nach Anspruch 1, wobei die Blätter eine Länge von 1,7 bis 2,1 cm und eine Breite von 0,9 bis 1,3 cm aufweisen.

3. Zusammensetzung nach Anspruch 1 oder 2, wobei das Ethanol 100%iges Ethanol ist.

4. Zusammensetzung nach einem der Ansprüche 1 bis 3, **dadurch gekennzeichnet, dass** der Bienenhonig die Qualität von Bioblütenhonig aufweist.

5. Verfahren zur Herstellung einer therapeutischen Zusammensetzung nach einem der Ansprüche 1 bis 4, mit den Schritten:
- Einbringen einer bestimmten Menge von Blättern der Pflanze *Salvia pisidica,* welche auf durchlässigem kalkhaltigen Boden gewachsen ist, in eine vorbestimmte Menge von zumindest 96%igem Ethanol zur Herstellung eines Suds;
- Stehenlassen des Suds, während mindestens 24 Stunden;
- Abfiltrieren der Blätter aus dem Sud; und
- Einrühren des Suds in verflüssigten Blütenhonig.

6. Verfahren nach Anspruch 5, **dadurch gekennzeichnet, dass** das Ethanol 100%iges Ethanol ist.

7. Verfahren nach einem der Ansprüche 5 oder 6, **dadurch gekennzeichnet, dass** frische Blätter verwendet werden.

8. Verfahren nach einem der Ansprüche 5 bis 7, **dadurch gekennzeichnet, dass** der Sud bei mindestens 25 Grad Celsius stehen gelassen wird.

9. Verfahren nach einem der Ansprüche 5 bis 8, **dadurch gekennzeichnet, dass** der Blütenhonig Bioqualität aufweist.

## Claims

1. A composition:
- an extract made with at least 96% ethanol from the leaves of Salvia pisidica grown on permeable calcareous soil;
- and
- Bee honey.

2. The composition according to claim 1, wherein the leaves have a length of 1.7 to 2.1 cm and a width of 0.9 to 1.3 cm.

3. The composition according to claim 1 or 2, wherein the ethanol is 100% ethanol.

4. The composition according to any one of claims 1 to 3, **characterized in that** the bee honey has the quality of organic blossom honey.

5. A process for the preparation of a therapeutic composition according to any one of claims 1 to 4, comprising the steps of:
- Incorporating a predetermined amount of leaves of the plant Salvia pisidica grown on permeable calcareous soil into a predetermined amount of at least 96% ethanol to produce a decoction;
- Leave the brew to stand for at least 24 hours
- Filtering the leaves from the brew; and
- Stir the decoction into liquefied blossom honey.

6. The process according to claim 5, **characterized in that** the ethanol is 100% ethanol.

7. A method according to one of claims 5 or 6, **characterized in that** fresh leaves are used.

8. The process according to any one of claims 5 to 7, **characterized in that** the brew is left to stand at at least 25 degrees Celsius.

9. The process according to one of claims 5 to 8, **characterized in that** the blossom honey is of organic quality.

## Revendications

1. Composition :
- un extrait, préparé avec au moins 96 % d'éthanol, de feuilles de Salvia pisidica ayant poussé sur un sol calcaire perméable ;
- ; et
- Miel d'abeille.

2. Composition selon la revendication 1, dans laquelle les feuilles ont une longueur de 1,7 à 2,1 cm et une largeur de 0,9 à 1,3 cm.

3. Composition selon la revendication 1 ou 2, dans laquelle l'éthanol est de l'éthanol à 100 %.

4. Composition selon l'une quelconque des revendications 1 à 3, **caractérisée en ce que** le miel d'abeille présente la qualité de miel de fleurs bio.

5. Procédé de préparation d'une composition thérapeutique selon l'une quelconque des revendications 1 à 4, comprenant les étapes consistant à :
- Introduction d'une quantité déterminée de feuilles de la plante Salvia pisidica, qui a poussé sur un sol calcaire perméable, dans une quantité prédéterminée d'éthanol à au moins 96 % pour produire une décoction ;
- Laisser reposer la décoction pendant au moins 24 heures
- Filtration des feuilles de la décoction ; et
- Incorporer la décoction dans du miel de fleurs liquéfié.

6. Procédé selon la revendication 5, **caractérisé en ce que** l'éthanol est de l'éthanol à 100 %.

7. Procédé selon l'une des revendications 5 ou 6, **caractérisé en ce que** l'on utilise des feuilles fraîches.

8. Procédé selon l'une quelconque des revendications 5 à 7, **caractérisé en ce que** le brassin est laissé au repos à une température d'au moins 25 degrés Celsius.

9. Procédé selon l'une des revendications 5 à 8, **caractérisé en ce que** le miel de fleurs est de qualité biologique.
